# EUROPÄISCHE PATENTANMELDUNG

(11) **EP 3 095 776 A1**
(43) Veröffentlichungstag der Anmeldung: **23.11.2016**
(21) Anmeldenummer: 15168404.0
(22) Anmeldetag: 20.05.2015
(51) Int. Cl.: C07C 41/30, C07C 43/23

(54) **Kupplung von einem Phenol und einem Aren unter Verwendung von Selendioxid**

(71) Anmelder: Evonik Degussa GmbH, 45128 Essen (DE)
(72) Erfinder: DYBALLA, Katrin Marie, 45657 Recklinghausen (DE); FRANKE, Robert, 45772 Marl (DE); FRIDAG, Dirk, 45721 Haltern am See (DE); MIRION, Michael, 55122 Mainz (DE); QUELL, Thomas, 55118 Mainz (DE); WALDVOGEL, Siegfried R., 55435 Gau-Algesheim (DE)

(57) **Zusammenfassung**

Verfahren zur Kupplung von einem Phenol und einem Aren unter Verwendung von Selendioxid, sowie neuartige Phenol-Aren-Derivate.

## Beschreibung

Die vorliegende Erfindung betrifft ein Verfahren zur Kupplung von einem Phenol und einem Aren unter Verwendung von Selendioxid, sowie neuartige Phenol-Aren-Derivate.

Als Biaryl bezeichnet man Verbindungen, bei denen zwei Arylgruppen über eine Einfachbindung miteinander verknüpft sind. Das einfachste Biaryl ist Biphenyl.

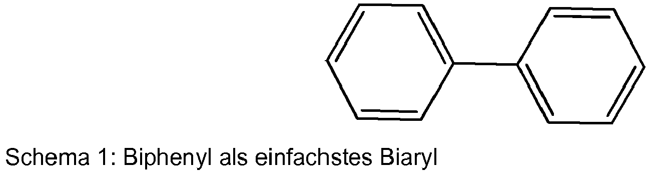

Bei den Phenol-Aren-Derivaten handelt es sich um Verbindungen, in denen die eine Aryleinheit eine OH-Gruppe trägt ("Phenol) und die andere Aryleinheit ("Aren") keine freie OH-Gruppe trägt. Alkoxygruppen, d.h. geschützte OH-Gruppen sind am Arenring jedoch zugelassen

Biphenole und Biaryle dienen als Synthesebausteine für katalytisch wirksame Substanzen und sind daher von industriellem Interesse. Biaryle stellen wichtige Bausteine für Flüssigkristalle, organische Vorrichtungen, Farbstoffe, Liganden für Metallkatalysatoren und finden sogar in medizinischen Bereichen Anwendungen, da diese Strukturen allgegenwärtig in biologisch aktiven, natürlich vorkommenden Produkten sind (vgl. a) R. Noyori, Chem. Soc. Rev. 1989, 18, 187; b) I. Cepanec, Synthesis of Biaryls, Elsevier, New York, 2004). Insbesondere die 2,2'-Biphenole können dafür verwendetet werden (vgl. WO 2005/042547). Diese finden vor allem als Ligandkomponenten für Katalysatoren Anwendung. Dabei kann das Biphenol beispielsweise als Ligandenbaustein in der enantioselektiven Katalyse verwendet werden (vgl. Y. Chen, S. Yekta, A. K. Yudin, Chem. Rev. 2003, 103, 3155-3211; J. M. Brunel Chem. Rev. 2005, 105, 857-898; S. Kobayashi, Y. Mori, J. S. Fossey, Chem. Rev. 2011, 11, 2626-2704).

Die direkte Kupplung ungeschützter Phenolderivate unter klassischen organischen Bedingungen ist bisher nur bei wenigen Beispielen gelungen. Hierfür wurden meist überstöchiometrische Mengen an anorganischen Oxidationsmitteln wie AlCl₃, FeCl₃, MnO₂ oder das organische 2,3-Dichlor-5,6-dicyano-1,4-benzochinon verwendet (vgl. G. Sartori, R. Maggi, F. Bigi, M. Grandi, J. Org. Chem. 1993, 58, 7271).

Alternativ werden solche Kupplungsreaktionen in einer mehrstufigen Sequenz durchgeführt. Hierbei werden Abgangsfunktionalitäten und oft giftige, komplizierte Übergangsmetallkatalysatoren, welche beispielsweise auf Palladium basieren, verwendet (vgl. L. Ackermann, Modern Arylation Methods, Wiley-VCH, Weinheim, 2009, X. Chen, K. M. Engle, D.-H. Wang, J.-Q. Yu, Angew. Chem. Int. Ed. 2009, 48, 5094-511, I. V. Seregin, V. Gevorgyan, Chem. Soc. Rev. 2007, 36, 1173-1193, G. Dyker, Handbook of C-H Transformations, Wiley-VCH, Weinheim, 2005).

Ein großer Nachteil der oben genannten Methoden zur Phenol-Kupplung ist die Notwendigkeit, in wasserfreien Lösemitteln unter Luftausschluss zu arbeiten. Häufig werden überstöchiometrische Mengen der entsprechenden Oxidationsmittel benötigt. Die Verknappung von Rohstoffen (z.B. Bor und Brom) führt zur steigenden Preisen, was zur Unwirtschaftlichkeit der Prozesse führt. Mehrstufige Synthesen bedingen die Verwendung verschiedener Lösemittel und mehrfache Aufreinigung bis zum gewünschten Produkt.

Die Synthese dieser Biphenole ist u.a. mittels elektrochemischer Verfahren möglich. Hierbei wurden Kohlenstoffelektroden wie Graphit, Glaskohlenstoff, Bor-dotierter Diamant (BDD) oder Edelmetalle wie Platin verwendet; vgl. WO2010139687A1 und WO2010023258A1. Nachteiteilig bei diesen elektrochemischen Verfahren sind die zum Teil teuren Apparaturen, die extra angefertigt werden müssen. Weiterhin ist eine Hochskalierung auf den Tonnenmaßstab, wie er in der Industrie üblicherweise benötigt wird, zum Teil sehr aufwändig und in einigen Fällen sogar unmöglich. Insbesondere eine Hochskalierung des Elektrodenmaterials (BDD) ist zurzeit noch nicht möglich.

Weiterhin erfordert die Darstellung durch elektrochemische Verfahren den Einsatz teils teurer Leitsalze, deren Wiederverwendbarkeit nicht gewährleistet werden kann. Der technische Aufwand elektrochemischer Reaktionen ist zudem immens. So ist die Herstellung großer Elektrodenflächen aus BDD nur begrenzt möglich und mit einem hohen Kostenaufwand verbunden. Bereits kleine Defekte an BDD-Oberflächen können zudem zu einer vollständigen Zerstörung der Elektroden führen.

Die Kupplung von Arenen und Phenolen zu den entsprechenden Phenol-Aren-Derivaten stellt ebenso wie die Kupplung von Arenen bzw. Phenolen zu den korrespondierenden Biaryl- bzw. Biphenolderivaten eine große Herausforderung dar, da diese Reaktionen oftmals weder regio- noch chemoselektiv sind.

Eine Aufgabe der folgenden Erfindung bestand darin, Verfahren bereitzustellen, bei dem Phenole und Arene miteinander gekuppelt werden können, ohne dass elektrochemische Verfahren notwendig sind und ohne dass mit brom- oder borhaltigen Abgangsfunktionalitäten an den OH-Gruppen gearbeitet werden muss.

Gelöst wird die Aufgabe durch ein Verfahren nach Anspruch 1.

Verfahren zur Herstellung von Phenol-Aren-Derivaten umfassend die Verfahrensschritte:
a) Zugabe eines Phenols zum Reaktionsgemisch,
b) Zugabe eines Arens aus der Gruppe der ein- und zweikernigen Arene,
c) Zugabe von Selendioxid zum Reaktionsgemisch,
d) Zugabe eines Lösemittels,
e) Erwärmen des Reaktionsgemisches, so dass das substituierte Phenol und das Aren, aus der Gruppe der ein- oder zweikernigen Arene, zu einem Phenol-Aren-Derivat umgesetzt werden.

In welcher Reihenfolge das Phenol, das Aren und das Selendioxid zu dem Reaktionsgemisch hinzuzugefügt werden, ist unerheblich. Grundsätzlich können auch weitere Komponenten in dem Reaktionsgemisch wie beispielsweise Lösemittel oder Säure enthalten sein.

Im Rahmen der vorliegenden Erfindung wird unter dem Begriff Areneinheit neben dem unsubstituierten Aryl und Phenyl als auch ein subsituiertes Aryl wie Phenyl aber auch Naphthyl verstanden. Unter dem Oberbegriff Aryle und Phenole sind in diesem Zusammenhang sowohl unsubstituierte, also auch substituierte Verbindungen zu verstehen, wie weiter unten ausgeführt. Die Substitution erfolgt hierbei am Benzolring.

Im Rahmen der vorliegenden Erfindung wird unter dem Begriff "Lösemittel" Verbindungen aus der Gruppe von Lösemittel Tetrahydrofuran, Ethylenglycoldimethylether, Bis(2-methoxyethyl)ether, Diethylether, Toluol, halogenierte Lösemittel oder halogenierte oder nicht halogenierte Säuren verstanden.

Der Zweck des Lösemittels besteht darin, eine Lösung, eine Vermischung und die Rührbarkeit der verschiedenen Komponenten miteinander zu gewährleisten.

In einer Variante des Verfahrens handelt es sich bei dem Phenol um eine Verbindung gemäß der allgemeinen Formel (I): 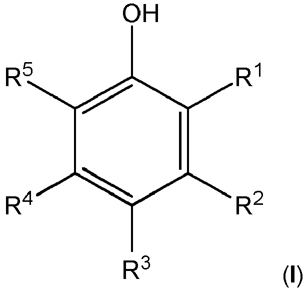 wobei R¹, R², R³, R⁴, R⁵ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen wobei die genannten Alkyl- und Arylgruppen substituiert sein können,
und mindestens eine der Reste R¹ oder R⁵ oder R² oder R⁴ gleich -H ist und bei dem einkernigen Aren handelt es sich um eine Verbindung gemäß der allgemeinen Formel (**II**): 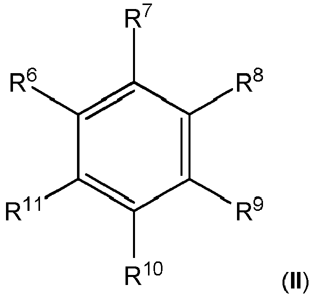 wobei R⁶, R⁷, R⁸, R⁹, R¹⁰, R¹¹ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen,
wobei die genannten Alkyl- und Arylgruppen substituiert sein können
und mindestens einer der Reste R⁶, R⁷, R⁸, R⁹, R¹⁰ oder R¹¹ gleich -H ist.

In einer Variante des Verfahrens sind R¹, R², R³, R⁴, R⁵ R⁶, R⁷, R⁸, R⁹, R¹⁰, R¹¹ jeweils unabhängig voneinander ausgewählt aus:
-H, -(C₁-C₁₂)-Alkyl, -O-(C₁-C₁₂)-Alkyl, -(C₆-C₂₀)-Aryl, -O-(C₆-C₂₀)-Aryl, -Cl,
wobei die genannten Alkyl- und Arylgruppen substituiert sein können,
und mindestens eine der Reste R⁶, R⁷, R⁸, R⁹, R¹⁰, R¹¹ gleich -H ist.

In einer Variante des Verfahrens sind R¹, R², R³, R⁴, R⁵ R⁶, R⁷, R⁸, R⁹, R¹⁰, R¹¹ jeweils unabhängig voneinander ausgewählt aus:
-H, -(C₁-C₁₂)-Alkyl, -O-(C₁-C₁₂)-Alkyl, -(C₆-C₂₀)-Aryl, -Cl,
wobei die genannten Alkyl- und Arylgruppen substituiert sein können,
und mindestens ein Rest R⁶, R⁷, R⁸, R⁹, R¹⁰, R¹¹ gleich -H ist

In einer Variante des Verfahrens sind die Rests R¹, R², R³, R⁴, R⁵ R⁶, R⁷, R⁸, R⁹, R¹⁰, R¹¹ jeweils unabhängig voneinander ausgewählt aus:
-H, -(C₁-C₁₂)-Alkyl, -O-(C₁-C₁₂)-Alkyl, -Cl,
und mindestens einer der Reste R¹, R⁵, R², R³ gleich -H ist,
und mindestens einer der Reste R⁶, R⁷, R⁸, R⁹, R¹⁰, R¹¹ gleich -H ist
wobei die genannten Alkylgruppen substituiert sein können.

In einer Variante des Verfahrens sind die Rests R¹, R², R³, R⁴, R⁵ R⁶, R⁷, R⁸, R⁹, R¹⁰, R¹¹ jeweils unabhängig voneinander ausgewählt aus:
-H, -(C₁-C₁₂)-Alkyl, -O-(C₁-C₁₂)-Alkyl,
und mindestens einer der Reste R¹, R⁵, R², R³ gleich -H ist,
und mindestens einer der Reste R⁶, R⁷, R⁸, R⁹, R¹⁰, R¹¹ gleich -H ist
wobei die genannten Alkylgruppen substituiert sein können.

In einer Variante des Verfahrens handelt es sich bei dem Phenol um eine Verbindung gemäß der allgemeinen Formel (**I**): 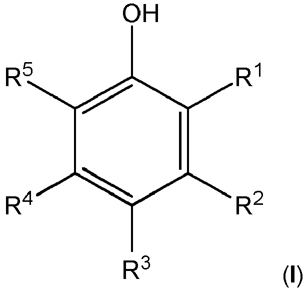 wobei R¹, R², R³, R⁴, R⁵ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen wobei die genannten Alkyl- und Arylgruppen substituiert sein können,
und mindestens eine der Reste R¹ oder R⁵ oder R² oder R⁴ gleich -H ist und bei dem zweikernigen Aren handelt es sich um eine Verbindung gemäß der allgemeinen Formel (**III**): 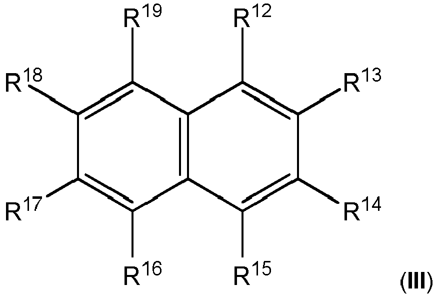 wobei R¹², R¹³, R¹⁴, R¹⁵, R¹⁶, R¹⁷, R¹⁸, R¹⁹ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen,
und mindestens einer der Reste R¹², R¹³, R¹⁴, R¹⁵, R¹⁶, R¹⁷, R¹⁸ oder R¹⁹ gleich -H ist,
wobei die genannten Alkylgruppen substituiert sein können.
(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.
(C₆-C₂₀)-Aryl und -(C₆-C₂₀)-Aryl-(C₆-C₂₀)-Aryl- können jeweils unsubstituiert oder durch einen oder mehrere gleiche oder verschiedene Reste substituiert sein, die ausgewählt sind unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.

Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrhydrofuryl, Tetrahydropyranyl, Menthyl und Dioxanyl.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, - CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂.

Substituierte -(C₆-C₂₀)-Arylgruppen und -(C₆-C₂₀)-Aryl-(C₆-C₂₀)-Arylgruppen sind vorzugsweise substituierte -(C₆-C₁₀)-Arylgruppen und -(C₆-C₁₀)-Aryl-(C₆-C₁₀)-Arylgruppen, insbesondere substituiertes Phenyl oder substituiertes Naphthyl oder substituiertes Anthracenyl. Substituierte -(C₆-C₂₀)-Arylgruppen tragen vorzugsweise eine oder mehrere z.B. 1, 2, 3, 4 oder 5 Substituenten, ausgewählt unter -(C₁-C₁₂)-Alkylgruppen, -(C₁-C₁₂)-Alkoxygruppen.

In einer Variante des Verfahrens sind R¹, R², R³, R⁴, R⁵ jeweils unabhängig voneinander ausgewählt aus:
-H, -(C₁-C₁₂)-Alkyl, -O-(C₁-C₁₂)-Alkyl, -(C₆-C₂₀)-Aryl, -O-(C₆-C₂₀)-Aryl, -Cl,
wobei die genannten Alkyl- und Arylgruppen substituiert sein können und mindestens einer der Reste R¹ oder R⁵ oder R² oder R⁴ gleich -H ist und es sich vorzugsweise bei dem zweikernigen Aren um eine Verbindung gemäß der allgemeinen Formel (III) handelt, wobei R¹², R¹³, R¹⁴, R¹⁵, R¹⁶, R¹⁷, R¹⁸, R¹⁹ jeweils unabhängig voneinander ausgewählt sind aus:
   -H, -(C₁-C₁₂)-Alkyl, -O-(C₁-C₁₂)-Alkyl, -(C₆-C₂₀)-Aryl, -O-(C₆-C₂₀)-Aryl, -Cl,
   wobei die genannten Alkyl- und Arylgruppen substituiert sein können,
   und mindestens ein Rest R¹², R¹³, R¹⁴, R¹⁵, R¹⁶, R¹⁷, R¹⁸ oder R¹⁹ gleich -H ist

In einer Variante des Verfahrens sind die Reste R¹, R², R³, R⁴, R⁵, R¹², R¹³, R¹⁴, R¹⁵, R¹⁶, R¹⁷, R¹⁸, R¹⁹ jeweils unabhängig voneinander ausgewählt aus:
-H, -(C₁-C₁₂)-Alkyl, -O-(C₁-C₁₂)-Alkyl, -Cl,
wobei die genannten Alkylgruppen substituiert sein können,
und mindestens einer der Reste R¹ oder R⁵ oder R² oder R³ gleich -H ist,
und mindestens einer der Reste R¹² , R¹³, R¹⁴, R¹⁵, R¹⁶, R¹⁷, R¹⁸ oder R¹⁹ gleich -H ist.

In einer Variante des Verfahrens sind die Reste R¹, R², R³, R⁴, R⁵, R¹², R¹³, R¹⁴, R¹⁵, R¹⁶, R¹⁷, R¹⁸, R¹⁹ jeweils unabhängig voneinander ausgewählt aus:
-H, -(C₁-C₁₂)-Alkyl, -O-(C₁-C₁₂)-Alkyl,
wobei die genannten Alkylgruppen substituiert sein können,
und mindestens einer der Reste R¹ oder R⁵ oder R² oder R³ gleich -H ist,
und mindestens einer der Reste R¹², R¹³, R¹⁴, R¹⁵, R¹⁶, R¹⁷, R¹⁸ oder R¹⁹ gleich -H ist.

Wird in dem erfindungsgemäßen Verfahren ein einkernigen Aren eingesetzt, so handelt es bei dem einkernigen Aren vorzugsweise um ein Phenylderivat.

Wird in dem erfindungsgemäßen Verfahren ein eingesetzt, so handelt es sich bei dem zweikernigen Aren vorzugsweise um ein Naphthylderivat.

Neben dem Phenol, dem Aren und dem Selendioxid kann das Reaktionsgemisch auch ein Lösemittel umfassen, wie beispielsweise Tetrahydrofuran, Ethylenglycoldimethylether, Bis(2-methoxyethyl)ether, Diethylether, Toluol, fluorierte Lösemittel oder organische Säuren. Durch die Zugabe des Lösemittels wird eine Verbesserung eine Lösung, Vermischung und/oder Rührbarkeit der verschiedenen Komponenten miteinander erreicht.

In einer Variante des erfindungsgemäßen Verfahrens wird ein fluoriertes Lösemittels zugegeben. In einer bevorzugten Ausführungsform des erfindungsgemäßen des Verfahrens wird eine Carbonsäure, bevorzugt Ameisensäure zugegeben.

In einer weiteren bevorzugten Ausführungsform des erfindungsgemäßen des Verfahrens wird als Lösemittel eine fluorierte Carbonsäure oder ein fluorierter Alkohol zugegeben.

In einer besonders bevorzugten Ausführungsform des erfindungsgemäßen Verfahrens wird als Lösemittel Trifluoressigsäure oder 1,1,1,3,3,3-Hexafluor-2-propanol und ganz besonders bevorzugt 1,1,1,3,3,3-Hexafluor-2-propanol zugegeben.

Ein besonderer Vorteil der hier beschriebenen Reaktionssysteme ist, dass diese nicht anfällig sind gegen feuchte Umgebungsluft, also einem Gemisch aus Wasserdampf, Sauerstoff und Stickstoff. Deswegen braucht nicht unter Luftabschluss gearbeitet zu werden, was die Durchführung der Reaktion erheblich vereinfacht und erst industriell praktikabel macht. Die Möglichkeit, das Verfahren in Anwesenheit feuchter Luft durchzuführen, ist daher von besonderem Interesse.

In dem erfindungsgemäßen Verfahren wird das Selendioxid in einem molaren Verhältnis bezogen auf die Summe des Phenols und des Arens zugegeben, welches bevorzugt in einem Bereich von 0,1 bis 2,0 liegt. Hierbei ist der Bereich von 0,25 bis 1,5 bevorzugt, und der Bereich von 0,4 bis 1,2 besonders bevorzugt.

Dass das Selendioxid in einer unterstöchiometrischen Menge eingesetzt werden kann, stellt einen weiteren Vorteil gegenüber der im Stand der Technik beschriebenen Reaktion mit anderen anorganischen Oxidationsmitteln wie beispielsweise AlCl₃, FeCl₃ oder MnO₂ dar.

In dem erfindungsgemäßen Verfahren wird das Reaktionsgemisch auf eine Temperatur in dem Bereich von 25 °C bis 120 °C bevorzugt von 30 °C bis 100 °C und besonders bevorzugt 30 °C bis 60 °C erwärmt.

Bei den hierbei angegebenen Temperaturen handelt es sich um die im Ölbad gemessenen Temperaturen. Das Erwärmen kann über einen Zeitraum im Bereich von 5 Minuten bis 24 Stunden erfolgen. Bevorzugt von 15 Minuten bis 12 Stunden und besonders bevorzugt von 15 Minuten bis 2,0 Stunden.

Neben dem Verfahren werden auch erfindungsgemäßen ein- und zweikernigen Aren-PhenolDerivate gemäß den Formeln **2**, **3**, **4** oder **5** beansprucht:

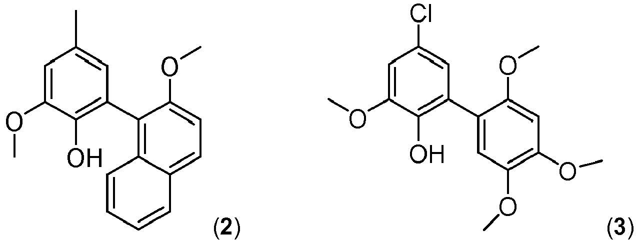

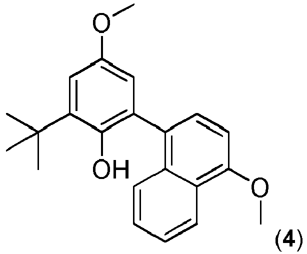

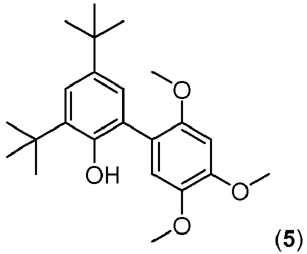

Nachfolgend wird die Erfindung anhand von Ausführungsbeispielen näher erläutert ohne diese darauf zu beschränken

### Analytik

### Chromatographie

Die präparativen flüssigkeitschromatographischen Trennungen via "Flashchromatographie" wurden mit einem Maximaldruck von 1.6 bar an Kieselgel 60 M (0.040-0.063 mm) der Firma *Macherey-Nagel GmbH & Co*, Düren durchgeführt. Die Trennungen ohne Druckbeaufschlagung wurden an Kieselgel Geduran Si 60 (0.063-0.200 mm) der Firma *Merck KGaA,* Darmstadt durchgeführt. Die als Eluentien verwendeten Lösemittel (Essigsäureethylester (technisch), Cyclohexan (technisch)) wurden zuvor destillativ am Rotationsverdampfer gereinigt.

Zur Dünnschichtchromatographie (DC) wurden PSC-Fertigplatten Kieselgel 60 F254 der Firma *Merck KGaA,* Darmstadt verwendet. Die Rf-Werte sind in Abhängigkeit vom verwendeten Laufmittelgemisch angegeben. Zur Anfärbung der DC-Platten wurde eine Cer-Molybdatophosphorsäure-Lösung als Tauchreagenz verwendet. Cer-Molybdatophosphorsäure-Reagenz: 5.6 g Molybdatophosphorsäure, 2.2 g Cer(IV)-sulfat-Tetrahydrat und 13.3 g konzentrierte Schwefelsäure auf 200 mL Wasser.

### Gaschromatographie (GC/GCMS)

Die gaschromatographischen Untersuchungen (GC) von Produktgemischen und Reinsubstanzen erfolgte mit Hilfe des Gaschromatographen GC-2010 der Firma *Shimadzu*, Japan. Es wird an einer Quarzkapillarsäule HP-5 der Firma *Agilent Technologies*, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma *Shimadzu*, Japan aufgenommen. Es wird an einer Quarzkapillarsäule HP-1 der Firma *Agilent Technologies*, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der lonenquelle: 200 °C) gemessen.

### Massenspektrometrie

Alle Elektrosprayionisation-Messungen (ESI+) wurden an einem QTof Ultima 3 der Firma *Waters Micromasses*, Milford, Massachusetts durchgeführt. EI-Massenspektren sowie die hochaufgelösten EI-Spektren wurden an einem Gerät des Typs MAT 95 XL Sektorfeldgerät der Firma *Thermo Finnigan*, Bremen, gemessen.

### NMR-Spektroskopie

Die NMR-spektroskopischen Untersuchungen wurden an Multikernresonanzspektrometern des Typs AC 300 oder AV II 400 der Firma *Bruker*, Analytische Messtechnik, Karlsruhe, durchgeführt. Als Lösemittel wurde CDCl3 verwendet. Die ¹H- und ¹³C-Spektren wurden gemäß dem Restgehalt an nicht deuteriertem Lösemittel nach der *NMR Solvent Data Chart* der Fa. *Cambridge Isotopes Laboratories*, USA, kalibriert. Die Zuordnung der ¹H- und ¹³C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Beispiele

### Allgemeine Arbeitsvorschriften

### AAV1: Arbeitsvorschrift zur Kupplung mittels Selendioxid

Ein Äquivalent der Phenolkomponente A wurde mit einem Äquivalent der Phenol/Arenkomponente B in 4-6 mL 1,1,1,3,3,3-Hexafluorisopropanol gelöst und mit 0,5 Äquivalenten Selendioxid versetzt. Das Gemisch wurde unter Rühren bis zum Sieden erhitzt. Nach einer Reaktionszeit von 60-75 Minuten wurde die Reaktionslösung filtriert, mit Ethylacetat verdünnt und mit Wasser gewaschen. Die organische Phase wurde abgetrennt, über Magnesiumsulfat getrocknet und das Lösemittel unter vermindertem Druck destillativ entfernt. Das so erhaltene Rohprodukt wurde säulenchromatographisch an Kieselgel 60 gereinigt.

### Beispiel 1

### 2-Hydroxy-2',3,4',5'-tetramethoxy-5-methylbiphenyl (1)

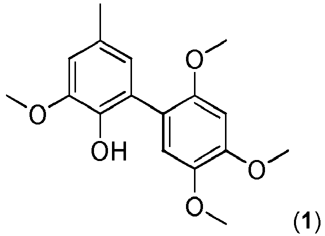

Die Reaktion wurde gemäß AAV1 mit 200 mg (1,45 mmol, 1,0 eq.) 4-Methylguajacol und 243 mg (1,45 mmol, 1,0 eq.) 1,2,4-Trimethoxybenzol in 4 mL HFIP und dem Zusatz von 80 mg (0,72 mmol, 0,5 eq.) Selendioxid durchgeführt. Die Reaktionszeit betrug eine Stunde. Nach Extraktion und Entfernung des Lösemittels wurde das erhaltene Produktgemisch mittels Säulenchromatographie an Kieselgel 60 im Laufmittel 5:1 (Cy:EE) gereinigt. Das Produkt wurde als blassgelbes, hochviskoses Öl erhalten.
Ausbeute: 139 mg (0,45 mmol, 32%)
¹H-NMR (400 MHz, CDCl₃): δ [ppm] = 2,33 (s, 3H), 3,81 (s, 3H) 3,86 (s, 3H), 3,91 (s, 3H), 3,94 (s, 3H), 5,98 (bs, 1 H), 6,65 (s, 1 H), 6,69 (s, 1 H), 6,71 (s, 1 H), 6,85 (s, 1 H)
¹³C-NMR (101 MHz, CDCl₃): δ [ppm] = 21,32, 5615, 56,29, 56,60, 57,44, 98,56, 111,37, 115,12, 118,70, 123,53, 125,34, 129,03, 141,02, 143,73, 147,89, 149,71, 150,47
HRMS (ESI, pos.mode): *m*/*z* ₅[M+Na⁺]: berechnet: 327,1208; gefunden: 327,1212.

### Beispiel 2

### 1-(2-Hydroxy-3-methoxy-5-methylphenyl)-2-methoxynaphthalen (2)

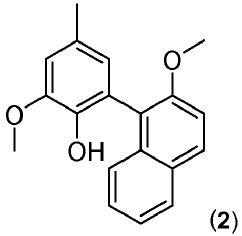

Die Reaktion wurde gemäß AAV1 mit 200 mg (1,45 mmol, 1,0 eq.) 4-Methylguajacol und 343 mg (2,17 mmol, 1,5 eq.) 1-Methoxynaphthalin in 5 mL HFIP und dem Zusatz von 96 mg (0,87 mmol, 0,6 eq.) Selendioxid durchgeführt. Die Reaktionszeit betrug 75 Minuten. Nach Extraktion und Entfernung des Lösemittels wurde das erhaltene Produktgemisch mittels Säulenchromatographie an Kieselgel 60 im Laufmittel 95:5 (Cy:EE) gereinigt. Das Produkt wurde als gelbes, hochviskoses Öl erhalten.
Ausbeute: 45 mg, (0,15 mmol, 11%)
¹H-NMR (400 MHz, CDCl₃): δ [ppm] = 2,36 (s, 3H), 3,89 (s, 3H), 3,95 (s, 3H), 5,37 (bs, 1 H), 6,65 (s, 1 H), 6,79 (s, 1 H), 7,33-7,36 (m, 2H), 7,40 (d, 1 H, *J=* 12 Hz), 7,46-7,49 (m, 1 H), 7,81-7,84 (m, 1 H), 7,91 (d, 1H, *J*= 12 Hz).
¹³C-NMR (101 MHz, CDCl₃): δ [ppm] = 21,39, 56,05, 57,11, 111,24, 113,96, 120,50, 122,21, 123,69, 124,46, 125,35, 126,50, 128,01, 129,10, 129,28, 129,60, 133,62, 141,51, 146,67, 154,89.
HRMS (ESI, pos.mode):*m*/*z* für C₁₉H₂₀O₃[M+H⁺]:berechnet:295,1333; gefunden:295,1334

### Beispiel 3

### 5-Chlor-2-hydroxy-2',3,3',6'-tetramethoxybiphenyl (3)

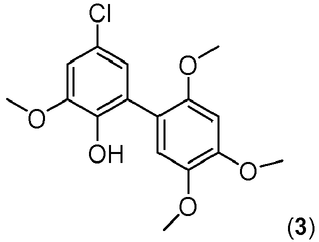

Die Reaktion wurde gemäß AAV1 mit 400 mg (2,52 mmol, 2,0 eq.) 4-Chlor-2-methoxyphenol und 313 mg (1,86 mmol, 1,0 eq.) 1,2,4-Trimethoxybenzol in 8 mL HFIP und dem Zusatz von 161 mg (1,45 mmol, 0,5 eq.) Selendioxid durchgeführt. Die Reaktionszeit betrug 75 Minuten. Nach Extraktion und Entfernung des Lösemittels wurde das erhaltene Produktgemisch mittels Säulenchromatographie an Kieselgel 60 im Laufmittel 5:1 (Cy:EE) gereinigt. Das Produkt wurde als braunes, hochviskoses Öl erhalten.
Ausbeute: 181 mg (0,56 mmol, 30%)
¹H-NMR (400 MHz, CDCl₃): δ [ppm] = 3,81 (s, 3H), 3,86 (s, 3H), 3,91 (s, 3H), 3,94 (s, 1H), 6,10 (bs, 1 H), 6,64 (s, 1 H), 6,82 (s, 1 H), 6,85 (d, 1H, *J*= 4 Hz), 6,88(d, 1 H, *J=* 4 Hz).
¹³C-NMR (101 MHz, CDCl₃): δ [ppm] = 56,27, 56,40, 56,61, 57,42, 98,44, 110,79, 114,79, 117,19, 122,88, 124,55, 126,49, 142,07, 143,80, 148,29, 149,81 , 150,40.
HRMS (ESI, pos.mode):*m*/*z* für C₁₆H₁₇O₅Cl [M+Na⁺]:berechnet: 347,0662, gefunden: 347,0663

### Beispiel 4

### 4-(3-tert-Butyl-2-hydroxy-5-methoxyphenyl)-1-methoxynaphthalene (4)

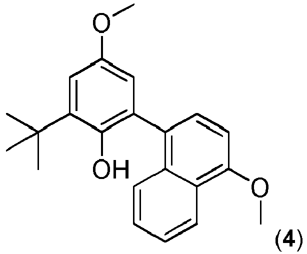

Die Reaktion wurde gemäß AAV1 mit 300 mg (1,66 mmol, 1,0 eq.) 4-Methoxy-2-*tert-*butylphenol und 263 mg (1,66 mmol, 1,0 eq.) 1-Methoxynaphthalin in 4,6 mL HFIP und dem Zusatz von 92 mg (0,83 mmol, 0,5 eq.) Selendioxid durchgeführt. Die Reaktionszeit betrug eine Stunde. Nach Extraktion und Entfernung des Lösemittels wurde das erhaltene Produktgemisch mittels Säulenchromatographie an Kieselgel 60 im Laufmittel 95:5 (Cy:EE) gereinigt. Das Produkt wurde als orange-braunes, hochviskoses Öl erhalten.
Ausbeute: 135 mg (0,4 mmol, 24%)
¹H-NMR (400 MHz, CDCl₃): δ [ppm] = 1,47 (s, 9H), 3,76 (s, 3H), 3,91 (s, 3H), 4,78 (bs, 1 H), 6,61 (d, 1 H,*J*= 4 Hz), 7,01 (d, 1 H, *J=* 4 Hz), 7,38-7,42 (m, 3H), 7,50-7,52 (m, 1 H), 7,84-7,87 (m, 1 H), 7,6 (d, 1 H, *J=* 8 Hz).
¹³C-NMR (101 MHz, CDCl₃): δ [ppm] = 29,69, 35,21, 55,73, 56,91, 112,90, 113,74, 114,16, 119,07, 123,23, 124,19, 125,20, 127,22, 128,15, 129,49, 130,61, 133,85 , 137,92, 146,54, 152,58, 154,72.

### Beispiel 5

### 3,5-Di-tert-butyl-2-hydroxy-2',4',5'-trimethoxybiphenyl (5)

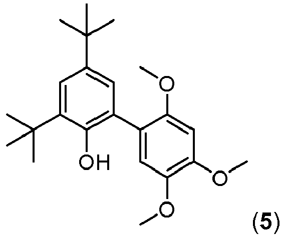

In einem 50 mL Rundkolben wurden 0.90 g 2,4-Di-*tert*-butylphenol (4.3 mmol) mit 3.63 g 1,2,4-Trimethoxybenzol (21.6 mmol) in 35 mL Hexafluorisopropanol gelöst und im 55 °C warmen Ölbad erhitzt. Nach 10 Minuten wurden 0.48 g Selendioxid (4.3 mmol) hinzugegeben. Nach 170 Minuten wurde die Reaktion durch Zugabe von 8 mL Wasser abgebrochen und das Hexafluorisopropanol unter vermindertem Druck abdestilliert. Der Rückstand wurde in 60 mL Ethylacetat aufgenommen und zweimal mit gesättigter Natriumchlorid-Lösung gewaschen. Die organische Phase wurde abgetrennt, über Magnesiumsulfat getrocknet und das Lösungsmittel unter vermindertem Druck destilliert. Überschüssiges 1,2,4-Trimethoxybenzol wurde im Kugelrohrofen entfernt (80 °C, 1*10⁻³ mbar). Das Rohprodukt wurde mittels Säulenchromatographie aufgereinigt. Dabei wurde ein Säulenautomat der Firma *BÜCHI-Labortechnik GmbH*, Essen verwendet. Die Säulenlänge betrug 32 cm und der Durchmesser 6 cm. Als Eluent wurde Cyclohexan/Essigsäureethylester verwendet, wobei mit einem Essigsäureethylester Gradienten gearbeitet wurde: 0% (über 5 min), 0-3 % (über 4 min), 3-6% (über 8 min), 6-12% (über 8 min), 12-24% (über 8 min), 24-48% (über 8 min), 48-100% (über 8 min). Die Pumpgeschwindigkeit betrug bei 100 mL/min.
Ausbeute: 0.727 g (1.9 mmol), 45%
GC: t_{R}(*hart*, HP-5) = 15.076 min
¹H-NMR: (400 MHz, CDCl₃) δ [ppm] = 1.35 (s, 9H), 1.48 (s, 9H), 3.84 (s, 3H), 3.88 (s, 3H), 3.96 (s, 3H), 6.20 (s, 1 H), 6.67 (s, 1 H), 6.88 (s, 1 H), 7.12 (d, *J*=2.5 Hz, 1 H), 7.37 (d, *J=* 2.5 Hz, 1 H).
¹³C-NMR: 29.98, 31.82, 34.49, 35.35, 56.38, 56.71, 57.35, 98.38, 116.06, 119.45, 123.72, 125.88, 126.14, 136.67, 142.34, 144.27, 149.64, 149.97, 150.11.

### Beispiel 6

### 5,6'-Dimethyl-2-hydroxy-2',3,3',4-tetramethoxybiphenyl (6)

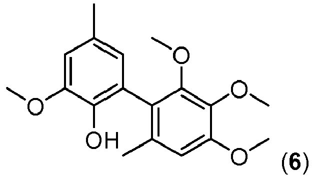

In einem 50 mL Rundkolben wurden 0.60 g 4-Methylguiacol (4.3 mmol) mit 6.04 g 3,4,5-Trimethoxytoluol (21.7 mmol) in 36 mL Hexafluorisopropanol gelöst und im 55 °C warmen Ölbad erhitzt. Nach 10 Minuten wurden 0.48 g Selendioxid (4.3 mmol) hinzugegeben. Nach 180 Minuten wurde die Reaktion durch Zugabe von 8 mL Wasser abgebrochen und das Hexafluorisopropanol unter vermindertem Druck abdestilliert. Der Rückstand wurde in 60 mL Ethylacetat aufgenommen und zweimal mit gesättigter Natriumchlorid-Lösung gewaschen. Die organische Phase wurde abgetrennt, über Magnesiumsulfat getrocknet und das Lösungsmittel unter vermindertem Druck destilliert. Überschüssiges 3,4,5-Trimethoxytoluol wurde im Kugelrohrofen entfernt (80 °C, 1*10⁻³ mbar). Das Rohprodukt wurde mittels Säulenchromatographie aufgereinigt. Dabei wurde ein Säulenautomat der Firma *BÜCHI-Labortechnik GmbH*, Essen verwendet. Die Säulenlänge betrug 32 cm und der Durchmesser 6 cm. Als Eluent wurde Cyclohexan/Essigsäureethylester verwendet, wobei mit einem Essigsäureethylester Gradienten gearbeitet wurde: 0% (über 5 min), 0-3 % (über 4 min), 3-6% (über 8 min), 6-12% (über 8 min), 12-24% (über 8 min), 24-48% (über 8 min), 48-100% (über 8 min). Die Pumpgeschwindigkeit betrug bei 100 mL/min.
Ausbeute: 0.586 g (1.8 mmol), 42%
GC: t_{R}(*hart*, HP-5) = 13.925 min
¹H-NMR: (400 MHz, CDCl₃) δ [ppm] = 2.06 (s, 3H), 2.32 (s, 3H), 3.68 (s, 3H), 3.89 (s, 3H), 3.91 (s, 3H), 5.44 (s, 1 H), 6.52 (d, *J=* 1.7 Hz, 1 H), 6.62 (s, 1 H), 6.69 (d, *J=* 1.7 Hz, 1 H). ¹³C-NMR: 20.19, 21.34, 55.97, 56.01, 61.04, 61.15, 109.04, 110.78, 123.24, 123.77, 124.03, 128.83, 132.84, 140.19, 140.86, 146.49, 151.77, 152.64.z
HRMS (ESI, pos.mode): *m*/*z* für[M+Na⁺]: berechnet: 341.1365, gefunden: 341.1376

Die Ergebnisse zeigen, dass das erfindungsgemäße Verfahren eine Syntheseroute darstellt, mit welcher gekuppelte Phenol-Aren-derivate selektiv hergestellt werden können.

## Patentansprüche

1. Verfahren zur Herstellung von Phenol-Aren-Derivaten umfassend die Verfahrensschritte:
a) Zugabe eines Phenols zum Reaktionsgemisch,
b) Zugabe eines Arens aus der Gruppe der ein- und zweikernigen Arene,
c) Zugabe von Selendioxid zum Reaktionsgemisch,
d) Zugabe eines Lösemittels,
e) Erwärmen des Reaktionsgemisches, so dass das substituierte Phenol und das Aren, aus der Gruppe der ein- oder zweikernigen Arene, zu einem Phenol-Aren-Derivat umgesetzt werden.

2. Verfahren nach Anspruch 1,
wobei es sich bei dem bei dem Phenol um eine Verbindung gemäß der allgemeinen Formel (**I**): 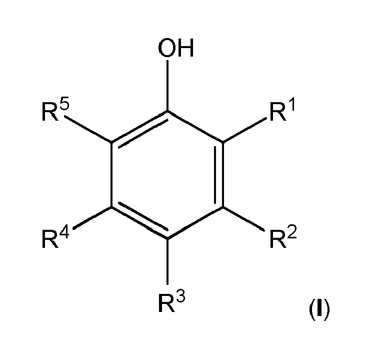 wobei R¹, R², R³, R⁴, R⁵ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen,
wobei die genannten Alkyl- und Arylgruppen substituiert sein können und mindestens R¹ oder R⁵ oder R² oder R⁴ gleich -H ist
und es sich bei dem einkernigen Aren um eine Verbindung gemäß der allgemeinen Formel (**II**) handelt: 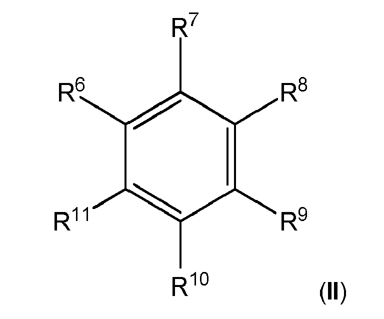 wobei R⁶, R⁷, R⁸, R⁹, R¹⁰, R¹¹ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen,
wobei die genannten Alkyl- und Arylgruppen substituiert sein können
und mindestens einer der Reste R⁶, R⁷, R⁸, R⁹, R¹⁰ oder R¹¹ gleich -H ist.

3. Verfahren nach Anspruch 2,
wobei R¹, R², R³, R⁴, R⁵ R⁶, R⁷, R⁸, R⁹, R¹⁰, R¹¹ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Cl,
wobei die genannten Alkyl- und Arylgruppen substituiert sein können,
und mindestens eine der Reste R⁶, R⁷, R⁸, R⁹, R¹⁰, R¹¹ gleich -H ist.

4. Verfahren nach Anspruch 2,
wobei R¹, R², R³, R⁴, R⁵ R⁶, R¹, R⁸, R⁹, R¹⁰, R¹¹ jeweils unabhängig voneinander ausgewählt sind aus:
-H, -(C₁-C₁₂)-Alkyl, -O-(C₁-C₁₂)-Alkyl, -(C₆-C₂₀)-Aryl, -Cl,
wobei die genannten Alkyl- und Arylgruppen substituiert sein können,
und mindestens ein Rest R⁶, R⁷, R⁸, R⁹, R¹⁰, R¹¹ gleich -H ist.

5. Verfahren nach Anspruch 2,
wobei die Rests R¹, R², R³, R⁴, R⁵ R⁶, R⁷, R⁸, R⁹, R¹⁰, R¹¹ jeweils unabhängig voneinander ausgewählt sind aus:
-H, -(C₁-C₁₂)-Alkyl, -O-(C₁-C₁₂)-Alkyl, -Cl,
und mindestens einer der Reste R¹, R⁵, R², R³ gleich -H ist,
und mindestens einer der Reste R⁶, R⁷, R⁸, R⁹, R¹⁰, R¹¹ gleich -H ist
wobei die genannten Alkylgruppen substituiert sein können.

6. Verfahren nach Anspruch 2,
wobei die Rests R¹, R², R³, R⁴, R⁵ R⁶, R⁷, R⁸, R⁹, R¹⁰, R¹¹ jeweils unabhängig voneinander ausgewählt sind aus:
-H, -(C₁-C₁₂)-Alkyl, -O-(C₁-C₁₂)-Alkyl,
und mindestens einer der Reste R¹, R⁵, R², R³ gleich -H ist,
und mindestens einer der Reste R⁶, R⁷, R⁸, R⁹, R¹⁰, R¹¹ gleich -H ist
wobei die genannten Alkylgruppen substituiert sein können.

7. Verfahren nach Anspruch 1,
wobei es sich bei dem bei dem Phenol um eine Verbindung gemäß der allgemeinen Formel (**I**) handelt: 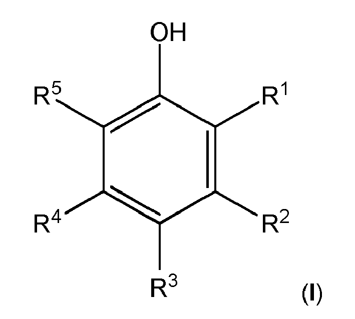 wobei R¹, R², R³, R⁴, R⁵ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen
wobei die genannten Alkyl- und Arylgruppen substituiert sein können
und mindestens einer der Reste R¹ oder R⁵ oder R² oder R⁴ gleich -H ist
und es sich bei dem zweikernigen Aren um eine Verbindung gemäß der allgemeinen Formel (**III**) handelt: 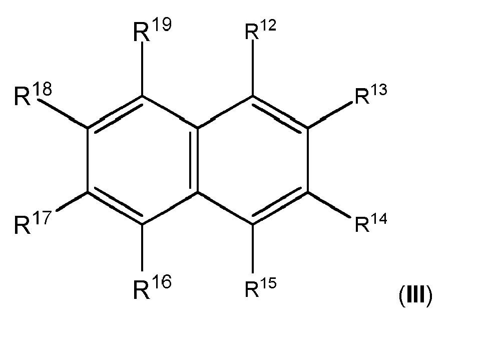
wobei R¹², R¹³, R¹⁴, R¹⁵, R¹⁶, R¹⁷, R¹⁸, R¹⁹ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Halogen,
und mindestens einer der Reste R¹², R¹³, R¹⁴, R¹⁵, R¹⁶, R¹⁷, R¹⁸ oder R¹⁹ gleich -H ist,
wobei die genannten Alkylgruppen substituiert sein können.

8. Verfahren nach Anspruch 7,
wobei R¹, R², R³, R⁴, R⁵ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Cl,
wobei die genannten Alkyl- und Arylgruppen substituiert sein können und mindestens einer der Reste R¹ oder R⁵ oder R² oder R⁴ gleich -H ist und es sich vorzugsweise bei dem zweikernigen Aren um eine Verbindung gemäß der allgemeinen Formel (III) handelt, wobei R¹², R¹³, R¹⁴, R¹⁵, R¹⁶, R¹⁷, R¹⁸, R¹⁹ jeweils unabhängig voneinander ausgewählt sind aus:
-H, -(C₁-C₁₂)-Alkyl, -O-(C₁-C₁₂)-Alkyl, -(C₆-C₂₀)-Aryl, -O-(C₆-C₂₀)-Aryl, -Cl,
wobei die genannten Alkyl- und Arylgruppen substituiert sein können,
und mindestens ein Rest R¹², R¹³, R¹⁴, R¹⁵, R¹⁶, R¹⁷, R¹⁸ oder R¹⁹ gleich -H ist

9. Verfahren nach Anspruch 7,
wobei die Reste R¹, R², R³, R⁴, R⁵, R¹², R¹³, R¹⁴, R¹⁵, R¹⁶, R¹⁷, R¹⁸, R¹⁹ jeweils unabhängig voneinander ausgewählt sind aus:
-H, -(C₁-C₁₂)-Alkyl, -O-(C₁-C₁₂)-Alkyl, -Cl,
wobei die genannten Alkylgruppen substituiert sein können,
und mindestens einer der Reste R¹ oder R⁵ oder R² oder R³ gleich -H ist,
und mindestens einer der Reste R¹², R¹³, R¹⁴, R¹⁵, R¹⁶, R¹⁷, R¹⁸ oder R¹⁹ gleich -H ist.

10. Verfahren nach Anspruch 7,
wobei die Reste R¹, R², R³, R⁴, R⁵, R¹², R¹³, R¹⁴, R¹⁵, R¹⁶, R¹⁷, R¹⁸, R¹⁹ jeweils unabhängig voneinander ausgewählt sind aus:
-H, -(C₁-C₁₂)-Alkyl, -O-(C₁-C₁₂)-Alkyl,
wobei die genannten Alkylgruppen substituiert sein können,
und mindestens einer der Reste R¹ oder R⁵ oder R² oder R³ gleich -H ist,
und mindestens einer der Reste R¹², R¹³, R¹⁴, R¹⁵, R¹⁶, R¹⁷, R¹⁸ oder R¹⁹ gleich -H ist.

11. Verfahren nach einem der Ansprüche 1 bis 10,
wobei das einkernige Aren ein Phenylderivat ist und das zweikernige Aren ein Naphthylderivat ist.

12. Verfahren nach einem der Ansprüche 1 bis 11,
wobei dem Reaktionsgemisch im Verfahrensschritt d) als Lösemittel eines aus der Gruppe von, fluorierten Alkoholen, fluorierten Carbonsäuren oder organischen Carbonsäuren zugegeben wird.

13. Verfahren nach einem der Ansprüche 1 bis 12,
wobei als Lösemittel 1,1,1,3,3,3-Hexafluor-2-propanol oder Trifluoressigsäure zugegeben wird.

14. Verfahren nach einem der Ansprüche 1 bis 13,
wobei das Selendioxid in einem molaren Verhältnis bezogen auf die Summe des Phenols und des Arens in einem Bereich von 0,1 bis 2,0 zugegeben wird.

15. Verbindungen gemäß den Formeln **2**, **3**, **4** oder **5**:
